# EUROPÄISCHE PATENTSCHRIFT

(11) **EP 2 793 677 B1**
(45) Veröffentlichungstag und Bekanntmachung des Hinweises auf die Patenterteilung: **06.04.2022**
(21) Anmeldenummer: 12821232.1
(22) Anmeldetag: 21.12.2012
(51) Int. Cl.: A61B 1/045, H04N 5/235, H04N 5/232, G02B 23/24, A61B 1/05, A61B 1/00, H04N 5/376, H04N 5/225

(54) **ENDOSKOPISCHE ANORDNUNG**
ENDOSCOPIC ARRANGEMENT
AGENCEMENT ENDOSCOPIQUE

(30) Priorität: 23.12.2011 CH 20472011
(43) Veröffentlichungstag der Anmeldung: 29.10.2014
(73) Patentinhaber: AWAIBA Consultadoria, Desenvolvimento e Comércio de Componentes Microelectrónicos, Unipessoal, Lda., Madeira (PT)
(72) Erfinder: WÄNY, Martin, CH-1400 Yverdon-les-Bains (CH)
(74) Vertreter: Epping - Hermann - Fischer
(86) Internationale Anmeldenummer: PCT/CH2012/000280
(87) Internationale Veröffentlichungsnummer: WO 2013/091124

(56) Entgegenhaltungen:
- EP-A2- 2 108 943
- WO-A1-2007/101360
- Martin Wäny ET AL: "Ultra small digital image sensor for endoscopic applications", Proc. of 2009 International Image Sensor Workshop, 26. März 2009 (2009-03-26), XP055035306, Bergen, Norway Gefunden im Internet: URL:http://www.imagesensors.org/Past Workshops/2009 Workshop/2009 Papers/044_paper_waeny_awaiba_endoscopy.pd f [gefunden am 2012-08-10]

## Beschreibung

### Technischer Bereich

Die Erfindung betrifft eine endoskopische Anordnung welche Pluraltät von Bildsensoren am distalen Ende aufweisst, wobei jeder der Bildsensoren angeordnet ist um einen eigenen Sensortakt zu erzeugen der von einer Steuerungselektronik am proximalen Ende der Endoskopanordnung beeinflusst werden kann.

Wenn im Rahmen dieser Offenlegung von "Bildphase" die Rede ist, so meint dies die Phase der Bildaufnahmesequenz mehrerer Bildsensoren zueinander oder die Phase der Bildaufnahme eines Bildsensors relativ zu einem Externen Takt. (z.B. zu einer gepulsten Belichtungsquelle). Dies ist nicht mit der Aufnahme eines "Phasenbildes" zu verwechseln. Bei letzterem werden von jedem Bildpunkt Information über die Phasenlage einer eintreffenden Frequenz auf diesen Bildpunkt ermittelt und als Bildhaftes Signal übertragen, während die Bildphase an und für sich im Zusammenhang dieser Erfindung nicht dazu dient Informationen über das Betrachtete Objekt zu übermitteln, aber wichtig ist um z.B. eine Pluralität von Bildsensoren so synchron betreiben zu können, dass alle dieser Bildsensoren mit einer einzelnen gepulsten Lichtquelle beleuchtet werden können.

### Stand der Technik

Für medizinische Untersuchungen, Eingriffe und Analysen werden oft Endoskope eingesetzt. Um durch natürlich vorhandene Körperöffnungen oder wenig traumatische Schnitte zum Operations- resp. Untersuchungsort zu gelangen werden Endoskope mit möglichst kleinem Durchmesser eingesetzt. Traditionell wurden solche Endoskope durch Bündel von Lichtleitern realisiert. Heutzutage wird mehr und mehr die CMOS Sensortechnologie eingesetzt, wobei ein miniaturisierter Bildsensor direkt am distalen Ende des Endoskopes angebracht wird und das Bild in Form eines elektrischen Signales übertragen wird.

Bildsensoren zum Einsatz am distalen Ende von medizinischen Endoskopen sind heutzutage meist so aufgebaut, dass sie weitgehend autonom funktionieren können und nur mittels einer Datenleitung sowie der Spannungsversorgung mit dem proximalen Ende verbunden sind. Das zuführen einer Taktleitung ist meistens nicht erwünscht aufgrund der dafür benötigten zusätzlich notwendigen Sensorkontakte und der zusätzlichen Signalleitungen für den Takt. Daher wird auf solchen Sensoren ein Oszillator integriert, der den Sensortakt autonom vorgibt. In der einfachsten Form wir ein digitaler Ringoszillator eingesetzt. Siehe den Artikel von M. Wäny et. Al. SPIE in der Zeitschrift "Photonics West" von Jan. 2009. Referenz :EI09-EI114-9_7249-32 Diese Art der Realisierung hat aber den Nachteil, dass der Sensortakt nur ungefähr bestimmt ist und aufgrund von Schwankungen im Herstellungsprozess sowie von Betriebsbedingungen z.B. der Betriebstemperatur stark beeinflusst wird.

Die Realisierung von frequenzstabilen Oszillatoren, mit einer Stabilität wie sie etwa bekannt ist von Quarz-Oszillatoren, ist in der reinen CMOS Technologie nicht möglich. Der durch einen solchen reinen CMOS Oscillator realisierte Takt weißt im Vergleich zu einem mit Hilfe eines Quarz Oszillator erzeugten Taktes grosse Schwankungen auf, insbesondere wenn die Sensor Temperatur variiert oder die Spannungsversorgung Schwankungen ausgesetzt ist. Im Artikel M. Wäny et al. "Ultra small digital image sensor for endoscopic applications" Proc. of 2009 International Image Sensor Workshop, 26. März 2009 (2009-03-26) XP55035306, Bergen, Norway werden Techniken beschrieben um einen miniaturisierten Bildsensor in Bezug auf die Beeinflussung seiner Funktionen, unter anderem auch in Bezug auf die Beeinflussung seiner Betriebsfrequenz durch die Umweltbedingungen, robuster zu gestalten. Es fehlen aber Möglichkeiten oder Methoden einen solchen autonom funktionierenden Sensor einem Extern vorgegebenen Takt anzugleichen. Für die Aufnahme der Bilder ist es allerdings oft erwünscht den Sensor synchron zu einem externen Takt zu betreiben, so dass die Bilddaten ohne Zwischenspeicherung an ein Bildausgabegerät, wie z.B. ein Video Monitor, ausgegeben werden können. Weiter gibt es Anwendungen, z.B. die stereoskopische Bildaufnahme, bei welchen es vorteilhaft ist, eine Pluralität von Bildsensoren synchron zueinander zu betreiben.

Die internationale Veröffentlichung WO 2007/101360 A1 betrifft einen miniaturisierter Bildaufnehmer für ein Endoskop kleinster Dimensionen der autonom funktioniert und seinen Sensortakt selbst generiert. Allerdings fehlen in der Veröffentlichung Methoden oder Möglichkeiten mehrere solcher Bildaufnehmer zueinander oder einen solchen Bildaufnehmer zu einem extern vorgegebenen Takt (z.B. von einer gepulsten Beleuchtung herrührend) synchron zu betreiben.

Die europäische Patentanmeldung EP 2 108 943 A2 beschreibt eine Vorrichtung zur Fluorszenz-Bildgebung die Leuchterzeugungsmittel umfasst. Allerdings sind die in dieser Veröffentlichung Beschriebenen Belichtungsquellen mittels eines Steuergerätes zur Bildfrequenz des Bildaufnehmers zu synchronisieren, es besteht keine Möglichkeit umgekehrt den Bildaufnehmer zum Takt der Beleuchtung zu synchronisieren. Weiter wird ebenfalls in dieser Veröffentlichung der Einsatz einer Pluralität von Bildaufnehmern zur Gewinnung von 3D Bilddaten erwähnt, allerdings bestehen keinerlei Möglichkeiten diese Bildaufnehmer untereinander zu synchronisieren. Dies wir insbesondere bei der Aufnahme von 3D Daten mit Objekten die sich im Bild bewegen leicht zu Artefakten und Fehlberechnungen führen, da die von einem sich im Bild bewegenden Körper die in der Zeitverschiebung der Bildaufnahmen zwischen den beiden Sensoren zurückgelegte Wegstrecke fälschlicherweise als Tiefeninformation wahrgenommen wird.

### Zusammenfassung der Erfindung

Da durch die beschränkten Platzverhältnisse an der Endoskop Spitze es wünschenswert ist, die Elektronik auf dem Bildsensor platzieren zu können, liegt der Erfindung die Aufgabe zugrunde die Elektronik auf dem Bildsensor soweit zu reduzieren um möglichst die gesamte Fläche des Sensors zur eigentlichen Bildaufnahme zu verwenden, wobei der Bildsensor einerseits autonom funktionieren soll, insbesondere ohne die Zuführung eines externen Taktsignals, andererseits aber synchron zu einem Externen Takt oder synchron in einer Pluralität von Bildsensoren untereinander die Einzelbilder gleichzeitig aufzeichnen und übertragen soll.

Die überraschende Lösung dieser Aufgabe besteht darin, dass die Steuerungselektronik Mittel aufweist den Sensortakt und/oder die Sensorbildrate und/oder die Sensorbildphase zu erkennen und einem Referenztakt anzugleichen.

Die Erfindung schlägt daher eine endoskopische Anordnung gemäß dem unabhängigen Anspruch 1 vor.

Der Sensor ermöglicht mit einem einfachen reinen CMOS Oszillator auf dem Bildsensor die Bilder synchron zu einem externen Takt aufzunehmen und zu übertragen und/oder eine Pluralität von Sensoren zueinander zu synchronisieren und die Bilddaten synchron auszugeben.

Die Erfindungsgemässe Sensorrealisierung ermöglicht die Funktionalität des Bildsensors zu erweitern ohne Sensorfläche für zusätzliche Elektronik zu benötigen.

Die endoskopische Anordnung weist einen oder eine Pluralität von Bildsensoren am distalen Ende auf, wovon jeder seinen eigenen Sensortakt selbst erzeugt, der von einer Steuerungselektronik am proximalen Ende der Endoskopanordnung beeinflusst werden kann, wobei die besagte Steuerungselektronik Mittel aufweist den Sensortakt und/oder die übertragene Sensorbildrate und/oder die Sensorbildphase jedes individuellen Sensors besagter Sensor Pluralität zu erkennen und dessen Grösse jeweils mit den entsprechenden Grössen der anderen Sensoren zu vergleichen und aller oder eines Teils der Sensoren der besagten Pluralität von Sensoren zueinander anzugleichen.

Weiter sind die besagten Bildsensoren vorteilhaft in CMOS (Complementary Metal Oxyde Semiconductor) Technologie ausgeführt.

Die endoskopische Anordnung gemäss der Erfindung ist so ausgelegt, dass die Beeinflussung des Sensortaktes über die Änderung der Sensor Versorgungsspannung oder durch Übermittlung von Konfigurationsdaten erfolgen kann.

Die endoskopische Anordnung gemäss der Erfindung ist vorteilhaft so ausgelegt, dass die Beeinflussung des Sensortaktes durch Übermittlung von Konfigurationsdaten über ein Bilddateninterface von besagter Steuerungselektronik zum Sensor erfolgen kann, und dass der Bildsensor besagte Konfigurationsdaten auswerten und die Frequenz der Sensor Takterzeugung entsprechend den Konfigurationsdaten ändern kann.

Die endoskopische Anordnung gemäss der Erfindung ist vorteilhaft so ausgelegt, dass die besagten Konfigurationsdaten multiplext über das Bilddateninterface übertragen werden können.

Die endoskopische Anordnung gemäss der Erfindung ist vorzüglich so ausgelegt, dass die besagten Konfigurationsdaten jeweils nach der Übertragung einer Bildzeile übertragen werden können.

Die endoskopische Anordnung gemäss der Erfindung ist vorteilhaft so ausgelegt, dass die besagten Konfigurationsdaten jeweils nach der Übertragung eines Bildes übertragen werden können.

Die endoskopische Anordnung gemäss der Erfindung ist vorteilhaft so ausgelegt, dass aus den Bilddaten der Pluralität von Bildsensoren mittels stereoskopischer Auswertung 3D Bilddaten gewonnen werden können.

Die endoskopische Anordnung gemäss der Erfindung ist vorzüglich so ausgelegt, dass besagte Bildsensoren mit einer gepulsten Lichtquelle synchronisiert werden können.

### Zusammenfassende Beschreibung der Zeichnungen

Nachstehend ist ein Ausführungsbeispiel der Erfindung anhand der Zeichnung beschrieben. Es zeigt:
Fig. 1 zeigt ein Blockschaltbild eines Endoskopes mit Abgleich der Bildsensortakterzeugung mittels Regelung der Sensorversorgungspannung,
Fig. 2 zeigt ein Blockschaltbild eines Bildsensors geeignet zur Integration an einer Endoskopspitze,
Fig. 3 zeigt einen Ringoszillator mit einer Chipspannungsversorgung zur Generierung eines Basischiptaktes, und
Fig. 4 zeigt ein Blockschaltbild eines Endoskopes mit Abgleich der Bildsensortakterzeugung mittels Konfigurationsinterface.

### Beste Art der Ausführung der Erfindung

Die in der Zeichnung dargelegten Realisierungsmöglichkeiten der endoskopischen Anordnung gemäss der Erfindung sind einzig als erläuternde Beispiele zu verstehen. Die Beispiele schränken nicht die Generalität der Erfindung ein.

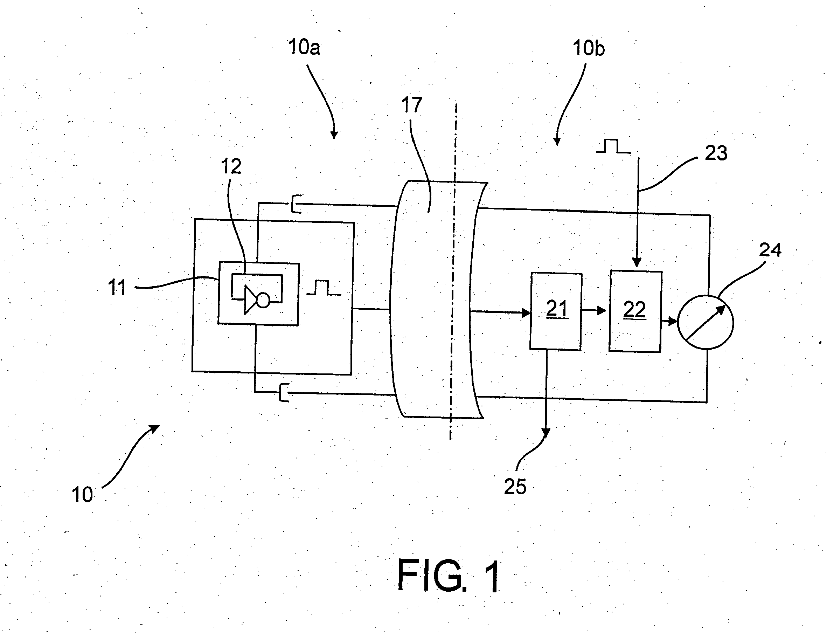

Fig. 1 zeigt die einfachste Realisierung einer erfingungsgemässen Endoskopanordnung 10 mit einer distalen Seite 10a und einer proximalen Seite 10b, die einen Bildsensor 11 aufweist, bei dem der Takt durch einen einfachen Ringoszillator 12 realisiert ist.

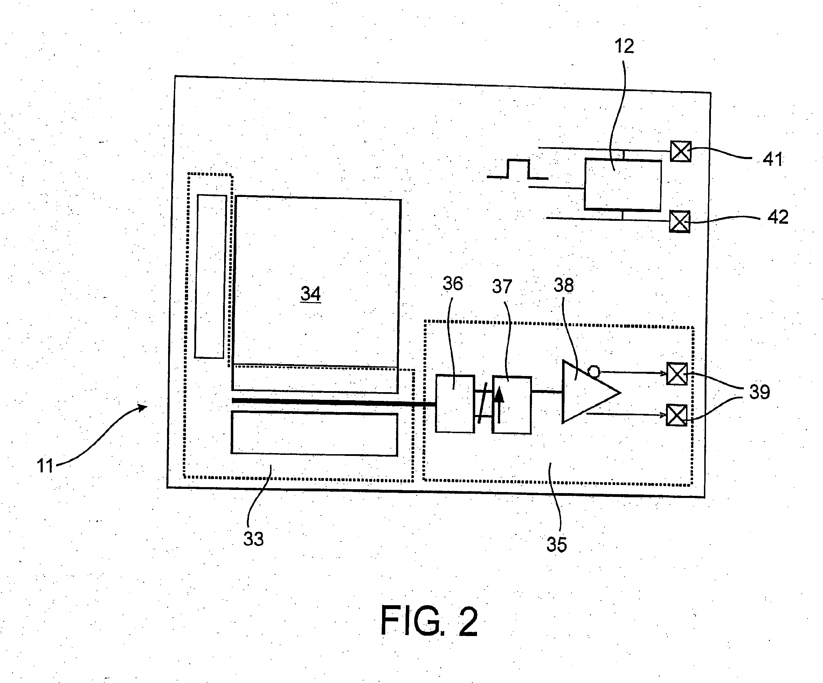

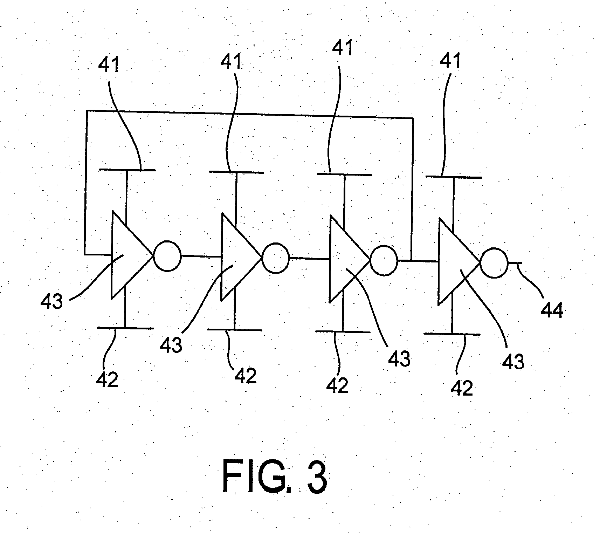

Der Bildsensor ist weiter in Fig. 2 erläutert und der Ringoscillator in Fig. 3. Der Bildsensor 11 ist über ein Daten- und Versorgungskabel 17 mit der Steuerungs und Auswerteelektronik im Proximalenteil verbunden. Vorzugsweise weist der in der Endoskopanordnung 10 eingesetzten Bildsensor 11 Möglichkeiten auf, den auf dem Bildsensor generierten Pixeltakt oder einen konstanten Teiler davon im Datenstrom zu übermitteln, zum Beispiel mittels Zeilen und Bildstart / Endsynchronisationspulsen. Weiter weist die Endoskopanordnung 10 im proximalen Teil oder in einem mit der Endoskopanordnung 10 verbundenen Steuer- oder Ausgabegerät Mittel 21 auf, die es ermöglichen den vom Bildsensor übermittelten Pixeltakt, oder den Zeilentakt, mindestens aber den Bildtakt, zu ermitteln, und Mittel 22, die es ermöglichen, diesen mit einem Referenztakt 23 zu vergleichen. Weiter weist die Endoskopanordnung 10 im proximalen Teil 10b ein Bilddaten Ausgabeinterface, (z.B. ein Video Monitor Anschluss) 25 auf. Ausserdem weist die Endoskopanordnung 10 im proximalen Teil 10b oder in einem mit der Endoskopanordnung 10 verbundenen Steuer- oder Ausgabegerät Mittel auf die Sensorversorgungspannung 24 so anzupassen, dass der Sensortakt bei einer detektierten Differenz des vom Sensor kommenden Taktes im Vergleich zum entsprechenden Referenztakt erhöht oder reduziert werden kann, je nachdem ob diese Differenz positiv oder negativ ausfällt. Nach bekanntem Wissenstand der Steuerungs- und Regelungstechnik ist die Korrektur der Bildsensorenversorgungspannung so ausgelegt, dass nach einer gewissen Anpassungszeit ein stabiler Sensortakt resultiert.

Fig. 2 stellt ein vereinfachtes Blockdiagramm der in der Endoskopanordnung 10 eingesetzten Bildsensors 11 dar. Der besagte Bildsensor 11 enthält eine Steuerung 33 zum Auslesen einer Bildpunktematrix 34 und nach bekanntem Stand der Technik eine geeignete Elektronik zur Übertragung der Bilddaten über einen Datenübertragungskanal 35, beispielhaft bestehend aus einem analog-Digitalwandler 36, Datenserialisierung 37 und differenziellem Signaltreiber 38 sowie einem differenziellen Dateninterface 39 . Der Sensortakt wird durch einen Ringoscillator 12, welcher proportional mit der Chipversorgungspannung 41 und 42 versorgt wird, generiert.

Fig. 3 erläutert detailliert einen Ringoszillator 12 welcher als Beispiel wie eine Schaltung aufgeführt ist, welche den Chiptakt erzeugt. Der Ringoscillator erzeugt einen periodischen Chiptakt dadurch, dass eine ungerade Anzahl von signalinvertierenden Schaltungselementen zu einem Ring verknüpft werden. In der beispielhaften Ausführung der Figur 3 sind drei digitale Inverter 43 zu einem Ring verknüpft. Jeder der Inverterblöcke ist zwischen der Chip Versorgungspannung 41 und dem Chip Erdpotential 42 versorgt. Unmittelbar nach Anlegen der Chip Versorgungspannung beginnt die Schaltung in Ihrer charakteristischen Eigenfrequenz zu schwingen. Eine einfache Verstärkerschaltung, in der Form eines weiteren Inverters 43 greift das Signal des Oszillatorrings ab und stellt es mit geringer Impedanz als Ausgangsignal zur Verfügung 44. Die Eigenfrequenz der Ringoszillators liegt höher, wenn er mit einer höheren Spannung versorgt wird, respektive bei einer geringeren Versorgungsspannung resultiert eine geringere Oszillationsfrequenz. Alternativ kann der Ringoszillator auch mit einer zur Versorgungsspannung proportionalen anderen Spannung versorgt werden.

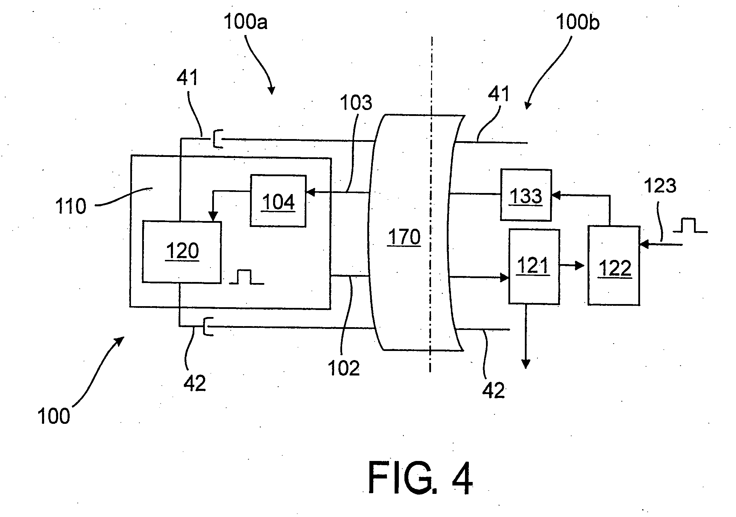

In einer alternativen Implementierung der Endoskopanordnung weist der Bildsensor Möglichkeiten auf, Konfigurationsdaten von der proximalen Kontroll- und Auswerteelektronik zu empfangen und mittels dieser Konfigurationsdaten die Sensortaktfrequenz graduell anzupassen. Der Bildsensor weist daher Mittel auf, den Sensortakt zu erhöhen oder zu reduzieren. Die Schrittweite der besagten Regelung des Bildsensortaktes ist nach bekannten Regeln der Steuerungstechnik so ausgelegt dass eine stabile Regelung der Bildphase und der Sensortaktfrequenz möglich ist. Insbesondere ist es möglich die Sensortaktfrequenz mittels kleiner kontinuierlicher Schritte soweit anzupassen bis eine Zielfrequenz und Zielphasenlage der Bildaufnahme erreicht ist. Die Konfigurationsdaten können nach bekanntem Stand der elektronischen Datenkommunikation sowohl über eine separate Konfigurationsleitung, oder multiplext über die Bilddatenleitungen übertragen werden. Die Übertragung der Konfigurationsdaten zur Anpassung der Sensortaktfrequenz kann dabei sowohl kontinuierlich wie zu bestimmten Zeiten, z.B. jeweils nach der kompletten Übertragung einer Bildzeile oder nach der Übertragung eines kompletten Bildes erfolgen. Je grösser das Intervall der Übermittlung besagter Konfigurationsdaten ist, je feiner sind die Frequenzanpassungsschritte auszulegen und je mehr Zeit wird benötigt werden um den Bildsensor auf einen Referenztakt oder eine Pluralität von Sensoren zueinander zu synchronisieren. Fig. 4 weist ein Blockschaltbild der Endoskopanordnung 100 dieser Variante auf und zeigt die für die Regelung des Sensortaktes notwendigen Funktionsgruppen schematisch auf:
Die Endoskopanordnung 100 besteht aus einem distalen Teil 100a und einem proximalen Teil 100b. Der distale und proximale Teil ist über ein Daten- und Versorgungskabel 170 miteinander verbunden. Der in der alternativen Endoskopanordnung eingesetzte Bildsensor 110 weist Mittel 104 auf, Kommunikationsdaten über ein Kommunikationsdaten Interface 103 zu empfangen und auszuwerten. Der Bildsensor 110 ist mit der Versorgungspannung 41 und dem Erdpotential 42 versorgt, welche in dieser Variante der erfindungsgemässen Endoskopanordnung fest eingestellt sind. Weiter weist der Bildsensor Mittel auf die Frequenz eines Oszillators 120 entsprechend den Steuerbefehlen der ausgewerteten Kommunikationssignale anzupassen. Nach bekannter Technik ist dies zum Beispiel durch einen mittels eines Digital-Analogwandlers kontrollierten "Spannungs kontrolliertem Oszillator" realisierbar. Weiter weist die alternative Endoskopanordnung 100 in der proximalen Auswerteelektronik 100b Mittel 121 auf, die es ermöglichen, den vom Bildsensor übermittelten Pixeltakt, oder den Zeilentakt mindestens aber den Bildtakt, zu ermitteln, und Mittel 122, die es ermöglichen, diesen mit einem Referenztakt 123 zu vergleichen. Weiter weist die Anordnung Mittel 133 auf, Kommunikationsdaten an den Bildsensor zurück zu übermitteln. Je nachdem ob der detektierte Sensortakt höher oder tiefer als der Referenztakt ist, wird nach bekannten Massgaben der Steuer- und Regelungstechnik über die Kommunikationsschnittstelle 103 und 104 der Oszillator 120 auf dem Bildsensor 110 so umkonfiguriert, dass nach einer gewissen Anpassungszeit ein stabiler Sensortakt resultiert, welcher dem Referenztakt in Frequenz und Phase gleich ist.

## Patentansprüche

1. Endoskopische Anordnung (10, 100) welche eine Steuerungselektronik und wenigstens einen Bildsensor (11 , 110) aufweist, wobei
- der Bildsensor am distalen Ende (10a, 100a) angeordnet und dazu eingerichtet ist, einen eigenen Sensortakt zu erzeugen, der von der Steuerungselektronik beeinflusst werden kann, und
- der Bildsensor (11 , 110) über ein Daten- und Versorgungskabel (17) mit der Steuerungselektronik und einer Auswertelektronik im proximalen Ende verbunden ist,
**dadurch gekennzeichnet, dass**
- die Steuerungselektronik am proximalen Ende (10b, 100b) der Endoskopischen Anordnung angeordnet ist und Mittel aufweist, den Sensortakt und/oder die übertragene Sensorbildrate und/oder die Sensorbildphase des am distalen Ende angeordneten Bildsensors zu erkennen und einem Referenztakt anzugleichen, so dass der wenigstens eine Bildsensor synchron zu einem nicht von diesem Sensor selbst vorgebenen Takt betrieben werden kann; wobei die Steuerungselektronik dazu ausgebildet ist, dass
- die Beeinflussung des Sensortaktes über eine Änderung der Sensorversorgungsspannung (24) mittels des Daten- und Versorgungskabels (17) erfolgt, oder
- die Beeinflussung des Sensortaktes über Empfang von Konfigurationsdaten mittels des Daten- und Versorgungskabels (17) erfolgt.

2. Endoskopische Anordnung nach Anspruch 1, umfassend eine Pluralität von Bildsensoren am distalen Ende, wobei
- jeder Bildsensor der Pluralität von Bildsensoren einen eigenen Sensortakt selbst erzeugt, der von der Steuerungselektronik am proximalen Ende der Endoskopanordnung beeinflusst werden kann,
- die Steuerungselektronik Mittel aufweist, den Sensortakt und/oder die übertragene Sensorbildrate und/oder die Sensorbildphase jedes der Bildsensoren der Pluralität von Bildsensoren zu erkennen und dessen Grösse jeweils mit den entsprechenden Grössen der anderen Bildsensoren zu vergleichen und aller oder eines Teils der Bildsensoren der Pluralität von Bildsensoren zueinander anzugleichen.

3. Endoskopische Anordnung nach Anspruch 1 oder 2, wobei der oder die Bildsensoren in CMOS (Complementary Metal Oxyde Semiconductor) Technologie ausgeführt sind.

4. Endoskopische Anordnung nach einem der Ansprüche 1 bis 3, wobei der oder die Bildsensoren jeweils einen Ringoszillator (12) aufweisen, der den Sensortakt realisiert.

5. Endoskopische Anordnung nach einem der Ansprüche 1 bis 4, die so ausgelegt ist, dass die Beeinflussung des Sensortaktes durch Übermittlung der Konfigurationsdaten über ein Bilddateninterface von besagter Steuerungselektronik zum Sensor erfolgen kann, und dass der Bildsensor Mittel aufweist, die Konfigurationsdaten auszuwerten und die Frequenz der Sensor Takterzeugung entsprechend den Konfigurationsdaten zu ändern.

6. Endoskopische Anordnung nach Anspruch 5, die so ausgelegt ist, dass die besagten Konfigurationsdaten multiplex über das Bilddateninterface übertragen werden können.

7. Endoskopische Anordnung nach Anspruch 6, wobei
- das Daten- und Versorgungskabel (17) eine separate Konfigurationsleitung aufweist, welche die Konfigurationsdaten über das Bilddateninterface überträgt, oder
- das Daten- und Versorgungskabel (17) Bilddatenleitungen umfasst, über die Konfigurationsdaten multiplex übertragen werden.

8. Endoskopische Anordnung nach einem der Ansprüche 1 bis 7, die so ausgelegt ist, dass die besagten Konfigurationsdaten jeweils nach der Übertragung einer Bildzeile übertragen werden können.

9. Endoskopische Anordnung nach einem der Ansprüche 1 bis 8, die so ausgelegt ist, dass die besagten Konfigurationsdaten jeweils nach der Übertragung eines Bildes übertragen werden können.

10. Endoskopische Anordnung nach Anspruch 2, wobei aus den Bilddaten der Pluralität von Bildsensoren mittels stereoskopischer Auswertung 3D Bilddaten gewonnen werden können.

11. Endoskopische Anordnung nach einem der Ansprüche 1 bis 10, die so ausgelegt ist, dass besagte Bildsensoren mit einer gepulsten Lichtquelle synchronisiert werden können.

## Claims

1. Endoscopic arrangement (10, 100) comprising
- control electronics and at least one image sensor (11, 110), the image sensor being arranged at the distal end (10a, 100a) and being set up to generate its own sensor clock which can be influenced by the control electronics, and
- the image sensor (11, 110) is connected to the control electronics and to an evaluation electronics in the proximal end via a data and supply cable (17),
**characterized in that**
- the control electronics are arranged at the proximal end (10b, 100b) of the endoscopic arrangement and have means for recognizing the sensor clock and/or the transmitted sensor image rate and/or the sensor image phase of the image sensor arranged at the distal end and for adapting it to a reference clock, so that the at least one image sensor can be operated synchronously to a clock not predetermined by this sensor itself; the control electronics being designed in such a way that
- the sensor clock is influenced by means of a change in the sensor supply voltage (24), by means of the data and supply cable (17), or
- the sensor clock is influenced by receiving configuration data by means of the data and supply cable (17).

2. Endoscopic arrangement according to claim 1, comprising a plurality of image sensors at the distal end, wherein
- each image sensor of the plurality of image sensors generates its own sensor clock itself, which can be influenced by the control electronics at the proximal end of the endoscope assembly,
- the control electronics comprise means for detecting the sensor clock and/or the transmitted sensor frame rate and/or the sensor frame phase of each of the image sensors of the plurality of image sensors and for comparing its size respectively with the corresponding sizes of the other image sensors and for aligning all or part of the image sensors of the plurality of image sensors with each other.

3. Endoscopic arrangement according to claim 1 or 2, wherein the image sensor(s) are implemented in CMOS (Complementary Metal Oxyde Semiconductor) technology.

4. Endoscopic arrangement according to any one of claims 1 to 3, wherein the image sensor or sensors each comprise a ring oscillator (12) which realizes the sensor clock.

5. Endoscopic arrangement according to any one of claims 1 to 4, which is designed such that the influencing of the sensor clock take place by transmitting configuration data via an image data interface from said control electronics to the sensor, and that the image sensor has means for evaluating the configuration data and changing the frequency of the sensor clock generation according to the configuration data.

6. Endoscopic arrangement according to claim 5, which is designed such that said configuration data be transmitted multiplexed via the image data interface.

7. Endoscopic arrangement according to claim 6, wherein
- the data and supply cable (17) comprises a separate configuration line which transmits the configuration data via the image data interface, or
- the data and supply cable (17) comprises image data lines via which configuration data is transmitted multiplexed.

8. Endoscopic arrangement according to any one of claims 1 to 7, which is designed such that said configuration data be transmitted each time after transmission of an image line.

9. Endoscopic arrangement according to any one of claims 1 to 8, which is adapted to transmit said configuration data each time after transmission of an image.

10. Endoscopic arrangement according to claim 2, wherein 3D image data be obtained from the image data of the plurality of image sensors by means of stereoscopic evaluation.

11. Endoscopic arrangement according to any one of claims 1 to 10, which is adapted to synchronize said image sensors with a pulsed light source.

## Revendications

1. Dispositif endoscopique (10, 100) qui comprend
- une électronique de commande et au moins un capteur d'image (11 , 110), le capteur d'image étant disposé à l'extrémité distale (10a, 100a) et étant conçu pour générer sa propre horloge de capteur, qui peut être influencée par l'électronique de commande, et
- le capteur d'images (11 , 110) est relié par un câble de données et d'alimentation (17) à l'électronique de commande et à une électronique d'évaluation dans l'extrémité proximale,
**caractérisé en ce que**
- l'électronique de commande est disposée à l'extrémité proximale (10b, 100b) du dispositif endoscopique et présente des moyens pour reconnaître la cadence de capteur et/ou le taux d'images de capteur transmis et/ou la phase d'images de capteur du capteur d'images disposé à l'extrémité distale et pour l'adapter à une cadence de référence, de sorte que le au moins un capteur d'images peut être exploité de manière synchrone à une cadence non prédéfinie par ce capteur lui-même ; l'électronique de commande étant conçue pour que
- l'influence sur la cadence du capteur s'effectue par le biais d'une modification de la tension d'alimentation du capteur (24) au moyen du câble de données et d'alimentation (17) ou
- l'influence sur la cadence du capteur s'effectue par la réception de données de configuration au moyen du câble de données et d'alimentation (17).

2. Dispositif endoscopique selon la revendication 1, comprenant
une pluralité de capteurs d'image à l'extrémité distale, dans laquelle
- chaque capteur d'image de la pluralité de capteurs d'image génère lui-même sa propre horloge de capteur, qui peut être influencée par l'électronique de commande à l'extrémité proximale de l'ensemble endoscopique,
- l'électronique de commande présente des moyens pour reconnaître la cadence du capteur et/ou la fréquence d'images transmises du capteur et/ou la phase d'images du capteur de chacun des capteurs d'images de la pluralité de capteurs d'images et pour comparer sa grandeur respectivement avec les grandeurs correspondantes des autres capteurs d'images et pour harmoniser entre eux tout ou partie des capteurs d'images de la pluralité de capteurs d'images.

3. Dispositif endoscopique selon la revendication 1 ou 2, dans lequel le ou les capteurs d'images sont réalisés en technologie CMOS (Complementary Métal Oxyde Semiconductor).

4. Dispositif endoscopique selon l'une des revendications 1 à 3, dans lequel le ou les capteurs d'images comportent chacun un oscillateur annulaire (12) qui réalise l'horloge du capteur.

5. Dispositif endoscopique selon l'une des revendications 1 à 4, qui est conçu de telle sorte que l'influence sur l'horloge du capteur peut être réalisée par la transmission des données de configuration par une interface de données d'image depuis ladite électronique de commande jusqu'au capteur, et que le capteur d'image comporte des moyens pour évaluer les données de configuration et modifier la fréquence de génération de l'horloge du capteur en fonction des données de configuration.

6. Ensemble endoscopique selon la revendication 5, conçu pour que lesdites données de configuration puissent être transmises multiplexées par l'interface de données d'image.

7. Ensemble endoscopique selon la revendication 6, dans lequel
- le câble de données et d'alimentation (17) comprend une ligne de configuration séparée qui transmet les données de configuration via l'interface de données d'image, ou
- le câble de données et d'alimentation (17) comprend des lignes de données d'image par lesquelles les données de configuration sont transmises de manière multiplexée.

8. Ensemble endoscopique selon l'une des revendications 1 à 7, agencé pour que lesdites données de configuration puissent être transmises après chaque transmission d'une ligne d'image.

9. Ensemble endoscopique selon l'une quelconque des revendications 1 à 8, agencé de manière à ce que lesdites données de configuration puissent être transmises après la transmission d'une image à la fois.

10. Dispositif endoscopique selon la revendication 2, dans lequel des données d'image 3D peuvent être obtenues à partir des données d'image de la pluralité de capteurs d'image au moyen d'une évaluation stéréoscopique.

11. Ensemble endoscopique selon l'une quelconque des revendications 1 à 10, conçu de telle sorte que lesdits capteurs d'image puissent être synchronisés avec une source de lumière pulsée.
